# EUROPÄISCHE PATENTANMELDUNG

(11) **EP 3 922 217 A1**
(43) Veröffentlichungstag der Anmeldung: **15.12.2021**
(21) Anmeldenummer: 21177408.8
(22) Anmeldetag: 02.06.2021
(51) Int. Cl.: A61F 2/844, A61F 2/958

(54) **ROHRFÖRMIGE VLIESSTRUKTUR ALS WIRKSTOFFTRÄGER ZUR ATRAUMATISCHEN BEHANDLUNG VON HOHLORGANEN SOWIE EIN VERFAHREN ZUR HERSTELLUNG**

(30) Priorität: 05.06.2020 DE 102020115094; 06.07.2020 DE 102020117801
(71) Anmelder: BVS - Best Vascular Solutions GmbH, 53173 Bonn (DE)
(72) Erfinder: Brohm-Schmitz-Rode, Andrea, 52070 Aachen (DE); Schmitz-Rode, Thomas, 52070 Aachen (DE)
(74) Vertreter: HGF

(57) **Zusammenfassung**

Erfindungsgemäß ist eine rohrförmige Vliesstruktur als Wirkstoffträger zur atraumatischen Behandlung von Hohlorganen (kurz "Hülse" genannt), insbesondere applizierbar über einen Ballonkatheter, sowie ein Verfahren zu deren Herstellung, wobei die Hülse in einem Ausgangszustand um eine Hülsenlängsachse gefaltet ist und in einem Endzustand zum Anliegen an einer Innenwandung eines Hohlorganes entfaltbar ist, wobei die rohrförmige Hülse aus zumindest ersten biodegradierbaren Polymer-Nano-Fasern ausgebildet ist und die Faltung der Hülse als Plissierung um eine Hülsenlängsachse gerichtet ist, wobei ein medizinischer Wirkstoff in die ersten Polymer-Nano-Fasern eingelagert und /oder in Zwischenräumen zwischen den Polymer-Nano-Fasern angeordnet ist, und wobei die ersten Polymerfasern derart ausgebildet sind, dass die Polymerfasern über einen Zeitraum von 2 Wochen bis 3 Monaten degradieren, sodass der Wirkstoff in diesem Zeitraum an eine Hohlorganwandung abgebbar ist.

## Beschreibung

Die vorliegende Erfindung betrifft eine rohrförmige Vliesstruktur (im Folgenden auch als "Hülse" bezeichnet) und ein System zur atraumatischen Behandlung von Hohlorganen sowie ein Verfahren zur Herstellung.

Zur Schienung von Engstellungen in menschlichen Hohlorganen, wie z.B. in Blutgefäßen, sind Stents bekannt. Ein Stent (Gefäßstütze) ist ein medizinisches Implantat, das in ein Hohlorgan einbringbar ist. Es handelt sich meist um ein rohrförmiges Gittergerüst aus Metall oder Kunststoff. Der Stent soll den betroffenen Abschnitt eines Hohlorganes stützen und auf diese Weise dauerhaft offenhalten.

In der Regel werden Stents mittels eines Katheters oder eines Ballonkatheters zum Implantationsort befördert. Hierfür kann der Stent im komprimierten Zustand auf einem Ballonkatheter angeordnet werden. Der Stent soll dabei einen möglichst geringen Außendurchmesser aufweisen, um beim Einführen in den menschlichen und/oder tierischen Körper das entsprechende Hohlorgan möglichst wenig zu beschädigen. Hierfür wird meistens mittels Radialkräften auf den Stent eingewirkt, sodass eine konzentrische Durchmesserreduktion erfolgt. Ist der Stent am Implantationsort platziert, wird der Ballonkatheter inflatiert, sodass eine konzentrische Aufweitung des Stents erfolgt.

Das Stentmaterial kann jedoch in manchen Fällen eine Gerinnselbildung (Thrombose) auslösen. Weiterhin kann es durch die mechanische Beanspruchung bei der Inflation des Ballonkatheters zu einer Verletzung der Hohlorganwandung des Hohlorganes kommen. Der Stent führt häufig langfristig zu einer chronischen Reizung. Auf diese Reizung reagiert die Hohlorganwandung mit einer Überproduktion von Wandzellen und sogenannter extrazellulärer Matrix (Hyperplasie). Eine solche Gefäßwandproliferation kann derart stark ausgeprägt sein, dass es zu einer Wiederverengung des Blutgefäßes (Restenose) kommt. Aufgrund der Thrombogenität der Risse in der Hohlorganwandung und auch des Stentmaterials wird häufig eine medikamentöse Antikoagulationsbehandlung durchgeführt, die eine Gerinnselbildung verhindern soll. Diese Therapie kann jedoch Nebenwirkungen haben. Daher wäre eine Reduktion der Medikation wünschenswert.

Weiterhin wird häufig versucht, die Gefäßwandproliferation durch sogenannte "Drug Eluting Stents" zu reduzieren. Derartige Stents sind in der Regel mit einem Polymer beschichtet, in das antiproliferative Wirkstoffe eingelagert sind bzw. sind diese Stents mit derartigen Wirkstoffen dotiert. Die Freisetzung der Wirkstoffe am Implantationsort reduziert die Überproduktion von Wandzellen. Jedoch verhindern diese Wirkstoffe bei manchen Patienten das Einwachsen des Stents in die Wandung des Gefäßes. Nach Beendigung der Antikoagulations-Medikation kann es zu sogenannten Spätthrombosen kommen, weil der Stent gar nicht oder nicht vollständig eingewachsen ist.

Um die vorstehenden Probleme zu vermeiden, werden neue Stentkonzepte verfolgt, zu denen auch sogenannte bioresorbierbare Stents zählen. Diese können aus biologisch abbaubaren Metalllegierungen, z. B. mit einem hohen Magnesiumanteil, oder aus biodegradierbaren Polymeren, z. B. Polylactid, ausgebildet sein. Hierbei ist vorgesehen, dass derartige Stents die Hohlorganwandung einige Monate stützen und anschließend durch körpereigene Substanzen biologisch abgebaut werden. Auf diese Weise soll auch die mechanische Reizung auf die Hohlorganwandung verringert werden und es soll zu weniger Restenose kommen. Ersten Studien zufolge kann aber auch bei biodegradierbaren Stents nicht auf die Beladung mit antiproliferativen Wirkstoffen verzichtet werden, weil die überschießende Gefäßwandreaktion bereits in den ersten Monaten nach der Implantation unterdrückt werden muss.

Derartige Vorrichtungen und Verfahren sind aus der DE 10 2012 007 640, der WO 02 076 700 A1, der US 5 443 495 A1, der DE 10 2006 020 687 A1, der US 2005 / 0 090 888 A1, der DE 2005 056 529, der US 2002 / 0 045 930 A1, der US 2005 /0 125 053 A1, der US 2008 / 0 262 594, der US 5,507,770 A, der US 6,059,823 A und der US 2010/249946 A1 bekannt.

Die Aufdehnung von Engstellen von Hohlorganen mittels Ballonkatheter ist fester Bestandteil der minimal-invasiven Therapie. Dies betrifft insbesondere die Blutgefäße. Im Rahmen der sog. endovaskulären Therapie werden Atherosklerose-bedingte Engstellungen und Verschlüsse von Blutgefäßen durch Ballonaufdehnung behandelt.

Typischerweise wird dazu ein Ballonkatheter unter Bildgebungskontrolle in das Gefäßsystem eingeführt. Nach Platzierung des Ballons im Bereich der zu behandelnden Läsion wird der Ballon unter hohem Druck aufgedehnt. Diese Standardbehandlung kann jedoch mit gravierenden Komplikationen verbunden sein. Durch die Ballonaufdehnung wird die Gefäßwand verletzt. Typischerweise entstehen Längsrisse in der Gefäßwand. Diese Verletzung kann in den ersten Tagen nach Behandlung eine Gerinnselanlagerung zur Folge haben. In den folgenden Tagen bis zu einem Zeitraum von etwa drei Monaten reagiert die Blutgefäßwand auf das Dilatationstrauma mit einer überschießenden Wandreaktion. Glatte Muskelzellen in der Gefäßwand werden durch das Trauma stimuliert und produzieren extrazelluläre Matrix ("intimale Hyperplasie"). Die damit verbundene Volumenzunahme in der Gefäßwand führt zu einer erneuten Ausbildung einer Verengung und wirkt damit dem Behandlungsergebnis entgegen. Auch die Implantation einer Gefäßstütze (Stent) zur dauerhaften Gefäßaufweitung kann dies nicht verhindern. Ein Stent ist ein Fremdkörper, der einerseits gerinnselbildend wirken kann, andererseits durch seine Steifigkeit die natürliche pulsatile Motilität des Stent-versorgten Gefäßabschnitts verhindert und so einen ständigen Reiz auf die Gefäßwand ausübt. Dieser Reiz kann wiederum zur überschießenden Produktion der Gefäßwandzellen mit Einwachsen des Gewebes durch die Stentstreben und damit zur Ausbildung einer erneuten Verengung führen.

Daher sind die heute üblicherweise implantierten Stents mit einem die Zellstimulation und Matrixproduktion reduzierenden ("antiproliferativen") Wirkstoff beschichtet. Im Falle einer alleinigen Ballondilatation ohne Stentimplantation werden heute überwiegend Ballonkatheter eingesetzt, deren Ballonhülle außen mit einem vergleichbaren Wirkstoff beschichtet ist, der bei Ballonaufdehnung in die Gefäßwand übertragen werden soll. Üblicherweise besteht die Beschichtung aus einem homogenen filmartigen Überzug der äußeren Ballonmembran, der den Wirkstoff eingebettet in ein Trägermaterial (Bindemittel, "Exipient") enthält. Das therapeutische Ziel ist die Übermittlung einer therapeutischen Wirkstoffdosis in die Gefäßwand für den kritischen Zeitraum von 6 Wochen bis max. 3 Monaten zur Unterdrückung der überschießenden Gefäßwandreaktion (Katsanos et al., J Am Heart Assoc 2018).

Insbesondere in der WO 2016/151035 A1 ist eine rohrförmige Hülse zur atraumatischen Behandlung von Hohlorganen beschrieben.

Aufgabe der vorliegenden Erfindung ist es, ein medizinisches Instrument zur atraumatischen Behandlung von Hohlorganen bereitzustellen, welches eine Alternative zu den aus dem Stand der Technik bekannten medizinischen Vorrichtungen, wie z.B. Stents, darstellt und breitere Anwendungsmöglichkeiten bietet.

Eine weitere Aufgabe der vorliegenden Erfindung ist es, ein verbessertes medizinisches Instrument zur atraumatischen Behandlung von Hohlorganen vorzusehen und insbesondere eine aus der WO 2016/151035 A1 bekannte rohrförmige Hülse weiterzubilden.

Zudem soll ein Verfahren zur Herstellung eines solchen medizinischen Instruments bereitgestellt werden.

Diese Aufgaben werden mit einer Vorrichtung gemäß dem Patentanspruch 1 und einem Verfahren gemäß Patentanspruch 11 gelöst. Vorteilhafte Ausgestaltungen davon sind in den Unteransprüchen angegeben.

Erfindungsgemäß ist eine rohrförmige Vliesstruktur als Wirkstoffträger (kurz "Hülse" genannt) zur atraumatischen Behandlung von Hohlorganen, insbesondere für eine Ballondilatation, wobei die Hülse in einem Ausgangszustand um eine Hülsenlängsachse gefaltet ist und in einem Endzustand zum Anliegen an einer Innenwandung eines Hohlorganes entfaltbar ist, wobei die rohrförmige Hülse aus zumindest ersten biodegradierbaren Polymer-Nano-Fasem ausgebildet ist und die Faltung der Hülse als Plissierung um eine Hülsenlängsachse gerichtet ist, wobei ein medizinischer Wirkstoff in die ersten Polymer-Nano-Fasem eingelagert und /oder in Zwischenräumen zwischen den Polymer-Nano-Fasern angeordnet ist, und wobei die ersten Polymerfasern derart ausgebildet sind, dass sie als langsam biodegradierbare Polymer-Nano-fasern (PL) über einen einstellbaren Zeitraum von 2 Wochen bis zu 3 Monaten degradieren, sodass der Wirkstoff in diesem Zeitraum an eine Hohlorganwandung abgebbar ist.

Unter dem Ausdruck "biodegradierbar" (bioabbaubar) wird im Rahmen der vorliegenden Erfindung verstanden, dass ein Kontakt der Polymer-Nano-Fasern bzw. des Polymers mit Körperflüssigkeit und einer Hohlorganwandung (speziell: Blut und Blutgefäßwand) einen Zersetzungs- bzw. Abbauprozess dieses Polymers induziert. Hierbei ist erfindungsgemäß vorgesehen, dass der medizinische Wirkstoff über denselben einstellbaren Zeitraum an eine Hohlorganwandung (speziell: Gefäßwandung) abgegeben wird. Somit lässt sich die Dauer der Therapie relativ genau einstellen.

Für die Hauptanwendung in Gefäßen, ist insbesondere vorgesehen, dass die ersten Fasern innerhalb von 2 Wochen bis zu 3 Monaten degradieren. Für die Anwendung der Kontrazeption können die Fasern derart ausgebildet sein, dass diese über einen langen Zeitraum von einem Jahr bis 5 Jahre insbesondere bis drei Jahre degradieren.

Bei bekannten Produkten zur atraumatischen Behandlung von Hohlorganen, insbesondere für eine Ballondilatation wird eine Außenfläche einer Ballonhülle mit einem Gemisch aus einem Trägerpolymer und einem Wirkstoff imprägniert, d.h. der Wirkstoff wird in Form multipler Teilchen (Nano- oder Mikropartikel, bzw."microreservoirs") appliziert. Somit gibt es keine zusammenhängende Hülse, die mittels eines Ballonkatheters appliziert bzw. abgeworfen wird.

In der US 2019/0046693 A1 ist bspw. eine homogene Beschichtung (homogeneous coating) vorgesehen, die eine Vielzahl von Mikroreservoiren bildet, die sich in dispergierter Form in einer lipophilen Matrix auf der Ballonhülle befinden und bei Ballonentfaltung an die Gefäßwand abgegeben werden. Eine rohrförmige Hülse im Sinne der vorliegenden Erfindung ist somit nicht vorgesehen.

Dadurch, dass die Hülse rohrförmig ausgebildet ist, weist diese eine Außenwandung und eine Innenwandung auf.

Die erfindungsgemäße rohrförmige Hülse wird auf einen Ballon eines Ballonkatheters aufgebracht. Durch Aufdehnung des Ballons im Hohlorgan (speziell: Blutgefäß) wird sie abgeworfen und haftet an der Hohlorganwand (speziell: Gefäßwand). Die Hülse wird somit durch die Ballonentfaltung im Hohlorgan (speziell: Blutgefäß) mit ihrer Außenwandung gegen die Hohlorganwand (speziell: Gefäßwand) gepresst. Dadurch entsteht während des Abwurfs ein flächiger Kontakt der gesamten Außenwandung der Hülse mit Körperflüssigkeit bzw. Blut, und nach Abwurf und Ballendeflation ein flächiger Kontakt der Innenwandung mit Körperflüssigkeit bzw. Blut, wobei die Außenwand in flächigem Kontakt mit der Hohlorganwand bzw. Gefäßwand steht. Da die Hülse mit einem medizinischen Wirkstoff beladen ist, diffundiert der Wirkstoff in die Hohlorganwand bzw. Gefäßwand. Die Wirkstoffabgabe erfolgt solange, bis die Hülse biologisch abgebaut worden ist.

Weiterhin kann die Hülse derart ausgebildet sein, dass durch eine "Liner-Funktion" die bei Ballondilatation entstehenden Längsrisse abgedeckt werden, um damit z. B. im Blutgefäß die Thrombogenität zu reduzieren. Einrisse in der Wandung des Hohlorganes bzw. Blutgefäßes, die durch die Expansion bzw. die Inflation des Ballonkatheters entstanden sind, können durch die entfaltete, die Läsion komplett abdeckende Hülse von direktem Kontakt mit Körperflüssigkeit bzw. Blut ausgeschlossen werden. Auf diese Weise wird im Bereich einer behandelten Gefäßläsion die Thrombogenität, d. h. die Neigung zur Blutgerinnselbildung vermindert.

Vorzugsweise kann die Faltung der rohrförmigen Hülse als Plissierung ausgebildet bzw. die Hülse plissiert sein. Unter Plissieren wird im Rahmen der vorliegenden Erfindung eine Faltung der Hülse in 2-4 (vorzugsweise drei, 3 x 120°) gleich große, auf dem Umfang gleichmäßig verteilte, in Axialrichtung verlaufende Falten verstanden, wobei die Falten gleichmäßig und gleichsinnig um die Längsachse des Ballonkatheters gewunden und an diesen angelegt werden. Durch das Plissieren ist es möglich, den Außendurchmesser der rohrförmigen Hülse im nicht-entfalteten Zustand zu reduzieren. Optional kann ein Adhäsiv auf die gefaltete Hülse aufgebracht sein, um die Faltung zu stabilisieren und damit den geringen Durchmesser beim Transport zum Implantationsort beizubehalten.

Die Faltung der Hülse ist um eine Hülsenlängsachse gerichtet, wobei die Faltung sowohl in als auch entgegen dem Uhrzeigersinn erfolgen kann. Das Falten und Plissieren erfolgt ähnlich dem in der WO 02 076 700 A1 anhand eines Ballonkatheteres beschriebenem Prinzip.

Die mit der Ballonaufdehnung implantierbare rohrförmige Hülse dient erfindungsgemäß als Träger für eine definierte Dosis eines medizinischen Wirkstoffes, der durch die rohrförmige Hülse sicher und zuverlässig in Kontakt mit einer Hohlorganwand bzw. Gefäßwand bringbar ist.

Durch die Vliesstruktur aus biodegradierbaren Polymer-Nano-Fasern wird eine therapeutisch wirksame Konzentration an Wirkstoff über einen vorbestimmten einstellbaren Zeitraum abgegeben, wobei dabei ein relativ konstanter Wirkstoffspiegel aufrechterhalten werden kann. Im Gegensatz zu bekannten beschichteten Ballonkathetern ohne rohrförmige Hülse findet kein rapider Abfall der Wirkstoffkonzentration an der Hohlorganwand nach der Ballonentfernung statt.

Im Gegensatz zu einem kurzfristigen Ballonkontakt eines direkt einem Wirkstoff beschichteten Ballonkatheters wird mittels der implantierten rohrförmigen Vliesstruktur und dem darin gebundenen Wirkstoff ein Abstrom eines großen Anteils an Wirkstoff in die Gefäßperipherie verhindert. Die damit verbundenen negativen systemischen Nebenwirkungen werden somit reduziert.

Unter einer Vliesstruktur wird im Rahmen der vorliegenden Erfindung eine Struktur aus Mikro- und/oder Nanofasern verstanden, die ein Vlies ausbildet. Die Wanddicke der Vliesstruktur ist kleiner 50 µm, vorzugsweise beträgt sie in etwa 10 bis 20 µm. Dabei soll die implantierte Hülse aufgrund ihrer nicht zu großen Eigensteifigkeit die natürliche Motilität der Hohlorganwand (speziell: Gefäßwand) nicht beeinträchtigen (also nicht so rigide sein wie ein herkömmlicher Stent).

Durch die Wirkstoffabgabe über den Abbau der biodegradierbaren Polymer-Nano-Fasern wird die therapeutisch erforderliche Zeitphase mit einem therapeutisch wirksamen Dosisspiegel abgedeckt. Die Biodegradation bzw. der Abbau der Fasern und somit die Wirkstoffabgabe soll erfindungsgemäß abgeschlossen sein, wenn die kritische Phase vorbei ist, sodass kein permanentes Implantat im Gefäß verbleibt.

Durch Einbettung des Wirkstoffes in eine lipophile Polymerkomponente der lokal fixierten Hülse wird die bei bekannten Systemen auftretende Auswaschung ("wash-off") von der Gefäßwand durch den Blutstrom und der Abstrom in die Peripherie deutlich reduziert.

Zwischen den Einzelfasern befinden sich Zwischenräume. Diese Zwischenräume bilden Mikrokompartimente im Sinne von WO 2016/151035 A1.

Erfindungsgemäß ist somit eine aus multiplen Polymer-Nano-Fasern gebildete rohrförmige Hülse auf einem Ballon eines Ballonkatheters anordbar und ist vorzugsweise zusammen mit dem Ballon des Ballonkatheters faltbar und aufdehnbar, wobei sich die Hülse bei Ballonaufdehnung vom Ballon löst und an die Hohlorganwand (speziell: Gefäßwand) gepresst wird, und nach Ballondeflatierung an der Hohlorganwand (speziell: Gefäßwand) haftet, und den Wirkstoff eingeschlossen in einer langsam degradierbaren Faserkomponente (PL) beinhaltet, die im Verlaufe ihres biologischen Abbaus (Degradation) den therapeutisch wirksamen Wirkstoffspiegel für einen kritischen Zeitraum im Kontakt zu der Hohlorganwand (speziell: Gefäßwand) liefert, und gleichzeitig auch die nach Ballondilatation entstandenen Längsrisse in der Hohlorganwand (speziell: Gefäßwand) abdichtet. Auf diese Weise lässt sich die Wirkstoffabgabe und auch deren Konzentration über die Zeit optimal auf den Anwendungsfall einstellen.

Die Hülse bleibt nach der Herstellung für den Zeitraum der Lagerung des Ballonkatheters auf der Ballonmembran bis zur Applikation komplett und vollständig, d. h., sie ist ein einziges, in sich konsistentes Bauelement, mit einer zusammenhängenden zylindrischen Innen- und Außenwandung, wobei zwischen den Fasern Mikrokompartimente ausgebildet sind.

Gemäß einer Ausführungsform behält die rohrförmige Hülse in etwa ihre ursprüngliche Dimensionierung nach einem ersten Körperflüssigkeits- bzw. Blutkontakt, weil eine schnell degradierbare Komponente aus Polymer-Nano-Fasern (PS) gering oder nicht vorhanden ist.

Die rohrförmige Hülse kann aus zumindest den ersten und zweiten, vorzugsweise biodegradierbaren, Polymer-Nano-Fasern ausgebildet sein. Es können auch weitere biodegradierbare oder nicht biodegradierbare Fasern vorgesehen sein.

Die zweiten Polymer-Nano-Fasern sind aus schnell degradierbaren Polymer-Fasern (PS) ausgebildet.

Vorzugsweise ist nur in das langsam degradierbare Polymer (PL) ein Wirkstoff eingelagert.

In die zweiten biodegradierbaren Polymer-Nano-Fasern oder in weitere Fasern kann ebenfalls ein medizinischer Wirkstoff eingelagert sein, wobei die zweiten Polymer-Nano-Fasern derart ausgebildet sind, dass die Fasern über einen Zeitraum von 1 Sekunde bis 2 Wochen, insbesondere 1 bis 3 Sekunden bis 10 Minuten, vorzugsweise 3 Sekunden bis 5 Minuten, degradieren, sodass der Wirkstoff in diesem Zeitraum an eine Hohlorganwandung abgebbar ist.

Vorzugsweise ist nur in das langsam degradierbare Polymer ein Wirkstoff eingelagert. In das schnell degradierende Polymer (PS) kann eventuell zusätzlich ein gerinnungshemmender Wirkstoff wie z.B. Heparin oder ein Antikoagulanz eingelagert sein.

Wenn zwei Polymerlösungen zur Herstellung der Vliesstruktur vorgesehen sind, die simultan (über parallele Düsenanordnung oder eine koaxiale Düsenanordung mit Zentralkanal und umgebendem Ringkanal, auch "Core-Shell-Nozzle" genannt) versprüht (Air Spraying) oder versponnen (Electrospinning) werden, kann z.B. Lösung 1, bestehend aus PL:PS 80:20 (Anteile in Volumenprozent), mit dem Wirkstoff Sirolimus vermischt werden, und Lösung 2, bestehend aus PL:PS 20:80, ohne Wirkstoff oder mit einem Antikoagulanz vermischt, appliziert werden.

In der entstanden Hülse ist dann ein sehr kleiner Teil Wirkstoff im schnell degradierbaren Polymer (zweites Polymer, PS), was ein Kauf genommen wird. Der Vorteil ist, dass der PS-Anteil die Faser adhäsiver macht und Schutzkolloid-Funktion haben kann.

Der Einfluss der Relation PS zu PL auf die Flake-Größe wird im Folgenden kurz beispielhaft aufgezeigt. Exemplarisch lässt sich für ein spezifisches PS und ein spezifisches PL folgende Abhängigkeit der Flake-Größe vom Mischungsverhältnis PS/PL feststellen:
PS: schnell degradierbares Polymer
PL: langsam degradierbares Polymer

| | Mittlere Flake-Größe bei Wasserkontakt in µm nach | | | | |
|---|---|---|---|---|---|
| PS/PL in Vol.% | 1 Minute | 1 Stunde | 1 Tag | 1 Woche | 1 Monat |
| 0/100 | >200 | 100 | 90 | 70 | 30 |
| 20/80 | >100 | 80 | 70 | 50 | 20 |
| 50/50 | 50 | 40 | 35 | 30 | 15 |
| 80/20 | 35 | 30 | 25 | 15 | 10 |

Bei einem PS-Anteil von 0 (100 Vol.-% PL) beträgt eine durchschnittliche bzw. mittlere Flake-Größe unmittelbar nach Wasser- oder Serumkontakt größer 200 µm und nach einer Woche in etwa 70 µm. Für ein PS-Anteil von 80 Vol.-% (20 Vol.-% PL) beträgt die mittlere Flake-Größe unmittelbar nach Wasser- oder Serumkontakt ca. 35 µm und nach einer Woche ca. 15 µm usw.

Die vorstehenden Ausführungen beschreiben nur beispielhaft die technische Wirkung der vorliegenden Erfindung für ein spezifisches PS und ein spezifisches PL.

Durch Änderung der Parameter Polymerart, Polymer-Kettenlänge (Molekulargewicht) und Art der Seitengruppen kann der Größenbereich für die mittlere Flake-Größe stark beeinflusst werden.

Das schnell degradierbare Polymer ist vorzugsweise sehr hydrophil, während das langsam degradierbare Polymer deutlich lipophilere Eigenschaften hat. Die Degradationsgeschwindigkeit hängt ab von der Polymerart (zum Beispiel bei PLGA - Poly(lactid-co-glycolid) durch das Verhältnis von GA zu LA (ein höherer Gehalt an LA bewirkt eine Reduktion der Hydrophilität und führt daher zu einer langsameren Auflösung), der Molekülkettenlänge (ein hohes Molekulargewicht bzw. eine längere Kettenlänge bewirkt eine langsamere Auflösung), und der Hydrophilität der Seitengruppen (z. B. wirken die Methyl-Seitengruppen in PLA hydrophob und verzögern damit die Auflösung, oder bei PLGA führt eine hydrophil wirkende Carboxylgruppe zu einer schnelleren Auflösung als eine Estergruppe).

Somit lässt sich die Degradationskinetik in Körperflüsigkeit oder im Blut einstellen, wobei diese neben der Geometrie (Faserdimensionen und Vliestextur) auch von der Art des Polymers, von dem Molekulargewicht und der Art der Seitenketten (hydrophil oder hydrophob) abhängt.

Die langsam und die schnell degradierbaren Polymer-Nano-Fasern können in der rohrförmigen Hülse in einem Verhältnis von 90 Vol-% zu 10 Vol-% bzw. von 80 Vol-% zu 20 Vol-% bzw. von 70 Vol-% zu 30 Vol-% bzw. von 60 Vol-% zu 40 Vol-% bzw. von 50 Vol-% zu 50 Vol-% bzw. von 40 Vol-% zu 60 Vol-% bzw. von 30 Vol-% zu 70 Vol-% bzw. von 20 Vol-% zu 80 Vol-% bzw. von 10 Vol-% zu 90 Vol-% vorgesehen sein.

Dadurch, dass gemäß einer bevorzugten Ausführungsform eine besonders schnell bioabbaubare Komponente PS vorgesehen ist, beginnt nach dem Abwurf der Hülse und der Anhaftung an der Hohlorganwand ein schneller Zerfall der Hülse in multiple Platten bzw. Plättchen bzw. Mikroflakes in einer insgesamt hülsenförmigen Anordnung.

Mit dieser Ausführungsform der implantierten Hülse, die durch Auflösung der schnell auflösenden Komponente eine schnellen Fragmentierung in Microflakes vorsieht, kann zusätzlich vermieden werden, dass kleine Seitenäste im Implantationssegment verlegt werden, weil sich im Abgangsbereich der Seitenäste durch die Seitenastperfusion keine durchgängig flächige Abdeckung ausbildet.

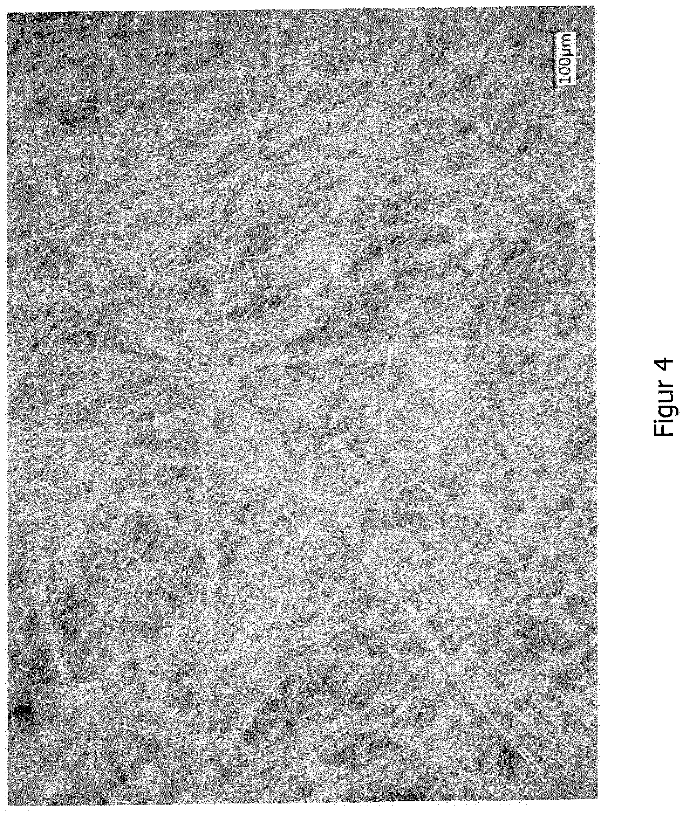

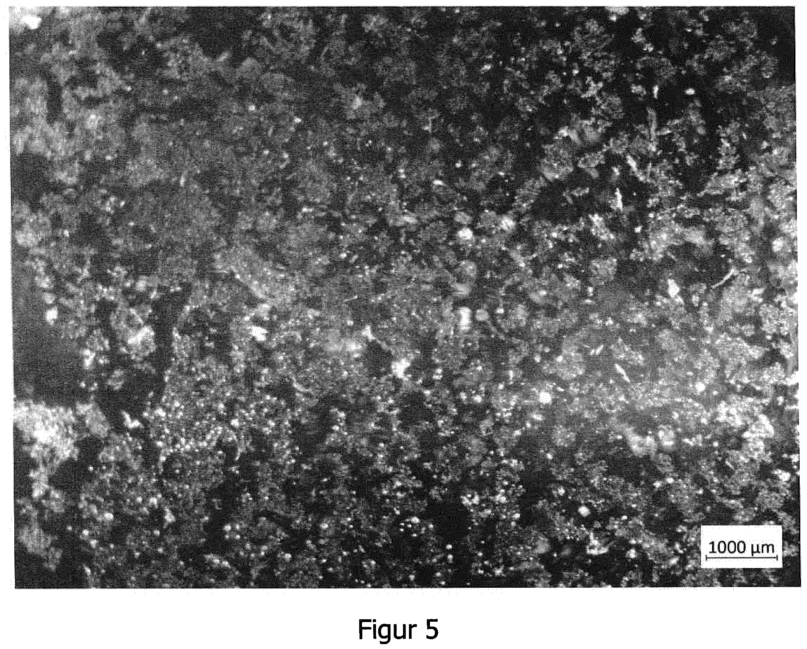

Universell einsetzbar ist insbesondere ein Verhältnis von langsam und schnell degradierbaren Polymer-Nano-Fasem in der rohrförmigen Hülse von 50 Vol-% zu 50 Vol-%. Die Geometrie der Fasern bei PS:PL 50:50 ist in Figur 4 dargestellt. Die in Figur 5 dargestellte Ausführungsform hat einen sehr kleinen PS-Anteil und zeigt die Auflösung in relativ große Fragmente ("Flakes") nach Wasserkontakt.

Bevorzugt für kleine Gefäße ist insbesondere ein Verhältnis von schnell zu langsam degradierbaren Polymer-Nano-Fasern in der rohrförmigen Hülse von PS = 70 Vol-% zu PL = 30 Vol-%. Ein Anteil von 70 Vol-% PS und mehr entspricht im Rahmen der vorliegenden Erfindung einem großen PS Anteil.

Bevorzugt für große Gefäße ist insbesondere ein Verhältnis von schnell zu langsam degradierbaren Polymer-Nano-Fasern in der rohrförmigen Hülse von PS = 30 Vol-% zu PL = 70 Vol-%. Ein Anteil von 30 Vol-% PS und weniger entspricht im Rahmen der vorliegenden Erfindung einem kleinen PS Anteil.

Je größer der Anteil an schnell degradierbaren Polymer-Nano-Fasern PS in der rohrförmigen Hülse ist, desto kleiner werden die Flakes. Daher eignet sich ein hoher Anteil an schnell degradierbaren Polymer-Nano-Fasem besonders für kleine Gefäße, da sich kleine Flakes besser an die Anatomie kleiner Gefäße anpassen können.

Somit zeichnet sich diese Ausführungsform dadurch aus, dass die schnelle Auflösung der entsprechenden Fasern (innerhalb weniger Sekunden bis zu 5 Minuten) einen Zerfall der vormals konsistent zylindrischen Hülse in multiple "Flakes" bzw. "Microflakes" bewirkt, die flach sind und der Hohlorganwand anhaften.

Dabei bestehen die resultierenden Flakes überwiegend aus der langsam degradierbaren Komponente, die den Wirkstoff enthält, während die schnell degradierbare Komponente durch Auflösung den Zusammenhalt der vormalig zusammenhängenden Hülse als ein einziges Teil aufhebt und den Zerfall der Hülse in multiple Teile ("Microflakes") induziert.

Bevorzugt hat ein einzelnes Flake eine flächige Ausdehnung von 10 bis 30 µm und eine Dicke von 5 bis 10 µm.

Die initiale Zerfallskinetik der Hülse in multiple Mikroflakes in einer tubulären Gesamtanordnung wird somit wesentlich bestimmt durch Anteil und Anordnung der schnell degradierbaren Komponente. Damit wird, abhängig von Anteil und Anordnung der schnell degradierbaren Komponente, nach Körperflüssigkeits- oder Blutkontakt, ein Gestaltübergang bewirkt, von zusammenhängend zylindrisch bzw. tubulär zu multiplen Microflakes, die zwar jeweils separat vorliegen, sich jedoch insgesamt in einer tubulären Gesamtanordnung befinden.

Als eine weitere Option wäre ein Gestaltübergang nach Blutkontakt von tubulärmikroporös (z.B. multiple Poren < 20 µm) zu tubulär makroporös (multiple Poren > 20 µm), d.h. große Poren, aber ohne den Zusammenhang der Hülse sofort aufzulösen, möglich. Das bedeutet, es findet keine sofortige Desintegration der Hülse mit Entstehung von multiplen Einzelteilchen (Mikroflakes) statt, sondern erst im weiteren zeitlichen Verlauf des Auflösungsprozesses.

Das biokompatible Polymer der Polymer-Nano-Fasern kann aus Polymeren auf Basis von Milchsäure (Polylactid, PLA), Glycolsäure (Polyglycolid, PGA) und ihrer Copolymere (Poly(lactid-co-glycolid), - PLGA), sowie Poly(ε-caprolacton), Polyethylenglycol, Polyethylenoxid, Polysebacinsäure, Poly(trime-thylencarbonat), Poly(ethylen-co-vinylacetat), Poly(1,5-dioxepan-2-on), Polyvinylpyrrolidon (PVP), Poly-p-dioxanone (PPDX) sowie deren Verbindungen und Copolymeren oder Mischungen daraus ausgebildet sein, wobei die Polymer-Nano-Fasern vorzugsweise einen Faserdurchmesser kleiner ein Mikrometer und vorzugsweise im Bereich von 300 bis 2000 nm und insbesondere im Bereich von 500 bis 1000 nm aufweisen. Die Polymer-Nano-Fasern können im Rahmen der vorliegenden Erfindung auch einen Durchmesser bis zu 3 Mikrometer aufweisen.

Die rohrförmige Hülse kann eine radiale Stützschicht aufweisen, wobei die radiale Stützschicht durch Polymer-Nano-Fasern mit höherer Festigkeit und/oder durch eine zusätzliche Polymerschicht ausgebildet ist.

Die radiale Stützschicht kann z.B. als ein lasergeschnittenes tubuläres, biodegradierbares Polymer-Halbzeug oder als eine mittels Melt Electrospinning Writing (MEW) gebildete Schicht ausgebildet sein.

Die Haftung an der Hohlorganwand kann bereits durch die radiale Eigensteifigkeit der Hülse (ohne Stützschicht) nach der Entfaltung verstärkt werden. Dies kann auch durch Kombination mit der mechanisch festeren radialen Stützschicht (eine Art Stützskelett) erreicht werden, die sich nur langsam abbaut.

Zumindest eine äußere Mantelwandung der Hülse kann adhäsive Eigenschaften aufweisen und/oder mit einer Beschichtung derart versehen ist, dass die Mantelwandung beim Entfalten an einer Innenwandung eines Hohlorganes anhaftet.

Vorzugsweise weist eine der beiden Polymerkomponenten klebrige/adhäsive Eigenschaften auf, wodurch das Haften an der Hohlorganwand begünstigt wird. Somit kann durch eine adhäsive Polymerkomponente die Haftung der Hülse an der Hohlorganwand verstärkt werden.

Für größere Hohlorgandurchmesser kann die Haftung an der Hohlorganwand auch durch die vorstehend aufgezeigte Möglichkeit zur Erhöhung der Radialstützkraft verbessert werden.

Bei Deflation des Ballons haftet die Hülse mit Ihrer Außenfläche an der Hohlorganwand (aufgrund des klebrig-adhäsiven Materialcharakters) und es entsteht durch komplette Ablösung der Hülse von der Ballonmembran ein flächiger Kontakt der gesamten Innenfläche der Hülse mit Körperflüssigkeit bzw. Blut.

Die gleichmäßige Ablösung von der Ballonmembran kann durch vorheriges Auftragen einer Trennschicht auf die Ballonmembran verbessert werden.

Die rohrförmige Hülse kann verschiedenste Durchmesser und Längen aufweisen, abgestimmt auf die Geometrie der zu behandelnden Hohlorganläsion. Beispielsweise kann eine Ausführung mit einem Durchmesser von 5 mm im expandierten Endzustand und einer Länge von 40 mm eine Wandstärke von 20-30 µm aufweisen.

Im Folgenden wird ein Verfahren zur Herstellung einer rohrförmigen Hülse zur atraumatischen Behandlung von Hohlorganen, insbesondere für eine Ballondilatation, beschrieben. Das Verfahren umfassend die folgenden Schritte:
Bereitstellen eines Gemisches aus zumindest einem in einem Lösungsmittel gelösten Polymer und einem medizinischen Wirkstoff,
schichtweises Auftragen des Gemisches auf einen zylindrischen Träger, um ein rohrförmiges Vlies aus Polymer-Nano-Fasern auszubilden,
Faltung des Vlieses nach Entfernung des Trägers und gleichsinnige Wicklung der Falten um eine zentrale Achse,
Aufbringung des gefalteten Vlieses auf die gefaltete und gewickelte Ballonmembran eines Ballonkatheters
   oder
schichtweises Auftragen des Gemisches zur Ausbildung eines rohrförmigen Vlieses aus Polymer-Nano-Fasern direkt auf den aufgedehnten Ballon eines Ballonkatheters, wobei der Ballon vorher mit einer Trennschicht versehen wurde,
und Faltung und gleichsinnige Wicklung des Vlieses zusammen mit der deflatierten Ballonmembran um den Ballonkatheterschaft.

Die einzelnen Polymer-Nano-Fasern verkleben während des Herstellvorgangs miteinander: Zusammenhalt, Formtreue und mechanische Eigenschaften ergeben sich aus der Gesamtkonsistenz der aneinanderhaftenden multiplen Einzelfasern.

Bei der Herstellung durch Sprühen (Air Spraying) oder durch Verspinnen in einem elektrischen Feld (Electrospinning) oder durch eine Kombination davon (Electrostatic Air Spraying) eignen sich als Lösungsmittel Substanzen mit ausreichend hohem Dampfdruck wie z. B. Chloroform, Aceton, Methanol, Heptan, Tetrahydrofuran, und weitere.

Ein erstes langsam degradierbares Polymer kann in einem ersten Lösungsmittel, bzw. einem Lösungsmittelgemisch aus zwei oder mehr Lösungsmitteln, gelöst sein, und ein zweites schnell degradierendes Polymer kann in dem ersten oder in einem zweiten Lösungsmittel, bzw. einem zweiten Lösungsmittelgemisch aus zwei oder mehr Lösungsmitteln, gelöst sein.

Dem Gemisch kann ein Schutzkolloid hinzugefügt sein, dass die Durchmischung verbessert und verhindert, dass sich der wasserunlösliche medizinische Wirkstoff abscheidet, wobei das Schutzkolloid vorzugsweise das schnelllösliche Polymer ist.

Der Anteil des Schutzkolloids kann 10 bis 20 Vol-% im Gemisch betragen und gleichzeitig später die Haftung der Mikroflakes an der Gefäßwand unterstützen bzw. verbessern.

Das Auftragen der Lösung auf den zylindrischen Träger oder direkt auf den Ballon kann durch Versprühen mit einem Luftstrahl (Air Spraying) oder durch Verspinnen in einem elektrischen Feld (Electrospinning) oder durch eine Kombination davon (Electrostatic Air Spraying) und/oder durch Tauchen in eine Lösung (Dip Coating) und/oder durch Aufbringen eines kontinuierlichen Schmelzestrangs (Melt Electrospinning Writing) erfolgen oder diskontinuierlich mittels 3D-Druck aufgetragen werden.

Normalerweise ist nur ein Verfahren zum Auftragen der Lösung vorgesehen. Jedoch, sofern technisch sinnvoll, können auch Kombinationen der genannten Verfahren möglich sein.

Auf den Träger kann vor dem Auftragen des Gemisches eine Trennschicht aufgetragen werden. Als Trennschicht eignen sich z.B. hämokompatible, gut wasserlösliche Polymere oder Zuckerverbindungen.

Der Wirkstoff kann auch gemischt in einer Polymer (langsam degradierend - PL)-Lösungsmittel-Lösung per Tauchverfahren (Dip Coating) außen auf das Vlies auftragen werden. Nach Verdampfen des Lösungsmittels lagert sich das Polymer PL mit dem Wirkstoff an die Fasern des Vlieses an, wodurch die Mikrokompartimente zwischen den Fasern teilweise gefüllt werden. Das Vlies kann dabei vorzugsweise rein aus schnell degradierendem Polymer PS oder einem PL/PS-Gemisch bestehen.

Vor dem Falten können intermittierende Längsschnitte im Verlauf der späteren Faltkanten in die Hülse eingebracht werden, um die Faltung zu erleichtern und den gewünschten Faltenverlauf auf der Mantelfläche und die gleichmäßige Verteilung der Faltkanten auf dem Umfang herbeizuführen. Diese Längsschlitze können vorzugsweise durch Laserschnitt erzeugt werden.

Der Träger kann ein zylindrischer Trägerkörper oder ein inflatierter Ballon sein.

Bevorzugt ist der direkte Auftrag auf einen Ballon. Dazu wird auf die aufgeblasene (inflatierte) Ballonhülle eines Ballonkatheters zunächst eine Trennschicht aufgetragen. Auf die Trennschicht wird dann die Hülse aufgetragen. Die rohrförmige Hülse kann zusammen mit dem Ballon des Ballonkatheters um einen Katheterschaft gefaltet werden.

Insbesondere ist erfindungsgemäß ein System zur atraumatischen Behandlung von Hohlorganen vorgesehen, das einen Ballonkatheter und die erfindungsgemäße rohrförmige Hülse umfasst.

Zudem kann bei diesem System auf der Hülse im Ausgangszustand eine äußere Schutzhülle angeordnet sein.

Durch eine solche folienartige Schutzhülle wird ein Blutkontakt vermieden und somit eine Thrombusbildung während des Einbringens der Hülse verhindert. Am Implantationsort kann die Schutzhülle dann durch Zurückziehen oder durch ein entfaltungsbedingtes Zerreißen entfernt werden. Darüber hinaus kann die Stabilität der Faltung der Hülse für den Transport auf dem Ballonkatheter durch eine adhäsive Oberflächenbehandlung der Außenfläche der Hülse unterstützt werden.

Weiterhin sind medizinische und therapeutische Verfahren zur Behandlung von Hohlorganen mit einer vorstehend aufgezeigten Vliesstruktur bzw. einem entsprechenden System vorgesehen.

Die Wirkstoff-Beladung des Wirkstoffträgers hängt ab vom Wirkort (Hohlorgan) und der Erkrankung. Der medizinische Wirkstoff kann einen oder mehrere der folgenden Stoffe umfassen.

Zur Reduzierung einer überschießenden Wandreaktion (intimale Hyperplasie) in Blutgefäßen, insbesondere in Arterien können als medizinscher Wirkstoff bspw. Limus-Derivate (z.B. Sirolimus) oder Paclitaxel (PTX) vorgesehen sein.

Limus-Derivate haben eine viel günstigere biologische Wirkung als Paclitaxel. Sie lassen sich jedoch schlecht mit herkömmlich beschichteten Ballons per einmaligem kurzen Ballonkontakt in die Gefäßwand übertragen, da sie deutlich schlechter anhaften als Paclitaxel-Kristalle, die in die Gefäßwand gespießt werden. Zudem benötigen sie wesentlich mehr Kontaktzeit für eine wirksame Übertragung in die Gefäßwand. Im Falle der abgeworfenen Hülse entspricht die Kontaktzeit mit der Gefäßwand der Lebendauer der Hülse, bis sie degradiert ist. Gleichzeitig ist die transferierte Hülse auch eine Gewähr für die transferierte Wirkstoffmenge (was bei einmaligem Ballonkontakt mit herkömmliche Systemen unwägbar ist) und sie hält den Wirkstoff besser am Wirkort (bei herkömmlicher Ballonübertragung wird ein großer Teil der übertragenen Partikel mit dem Blutstrom in die Peripherie abgeschwemmt).

Für den Einsatz im Blutgefäß soll die implantierte Hülse somit insbesondere als Träger für Antiproliferativa wie Limus-Derivate dienen und damit deren komplette Übertragung mit einem längerfristigen Kontakt zur Gefäßwand gewährleisten.

Bei einer Applikation der Hülse als Eileiter-Wirkstoffträger mit kontrazeptiver Wirkung können als medizinsche Wirkstoffe langzeitstabile Depot-Gestagene, wie z.B. Etonogestrel, Levonorgestrel oder ein geeignetes Antiprogesteron, wie z.B. Mifepriston oder ein Spermizid, wie z.B. Nonoxinol 9 vorgesehen sein. Grundsätzlich ist auch eine Applikation mit einem kontrazeptiven Wirkstoff im Bereich des Ductus deferens (Samenleiter) möglich.

Zur Therapie von Karzinomen in Hohlorganen, z.B. in den Gallenwegen, können für die Hülse als medizinsche Wirkstoffe Zytostatika wie z.B. Mitomycin, Capecitabin oder Methotrexat (MTX) vorgesehen sein. Weitere mögliche Applikationen der Hülse umfassen weitere Hohlorgane wie den Pankreasgang, die ableitenden Harnwege, Lymphgefäße, wie den Ductus thoracicus, das Tracheobronchialsystem, den Ductus nasolacrimalis, die Tuba Eustachii oder auch den Gastrointestinaltrakt.

Im Folgenden wird die Erfindung anhand der in den Figuren dargestellten Ausführungsbeispiele näher beschrieben. Diese zeigen in:
- Figur 1: eine schematische Darstellung einer entfalteten rohrförmigen Vliesstruktur ("Hülse"), angeordnet auf einem inflatierten Ballonkatheter, gemäß der vorliegenden Erfindung,
- Figur 2: eine schematische Darstellung der Vliesstruktur nach Abwurf vom Ballonkatheter, wobei die Hülse Minuten bis Stunden Blutkontakt hatte, verbunden mit dem Beginn der Desintegration in multiple Mikroflakes,
- Figur 3: eine schematische Darstellung der Vliesstruktur nach einigen Tagen bis Wochen, sodass sich multiple Mikroflakes in einer zylindrischen Gesamtkonfiguration ausgebildet haben,
- Figur 4: ein Mikroskopiebild der Fasertextur bei einem Verhältnis von PS zu PL von 50:50 Vol-% in einer Lösung, aufgetragen durch ein Sprühverfahren,
- Figur 5: ein Mikroskopiebild der Auflösung nach einem Tag, wobei die Größe der Flakes einer Vliesstruktur mit einem sehr geringen Anteil an schnell degradierbaren Polymer entspricht,
- Figur 6: eine schematische Darstellung einer atherosklerotischen Engstelle in einer Arterie,
- Figur 7: eine schematische Darstellung des erfindungsgemäßen Systems während einer Ballondilatation und dem damit verbundenen Abwurf der Hülse,
- Figur 8: eine schematische Darstellung der Vliesstruktur, angeordnet an einer Gefäßwand nach einer Ballonentfernung, und
- Figur 9: eine schematische Darstellung der Vliesstruktur einige Stunden bis Tage nach Abwurf im Zustand der zunehmenden Degradierung und Desintegration, verbunden mit dem Zerfall in multiple Flakes.

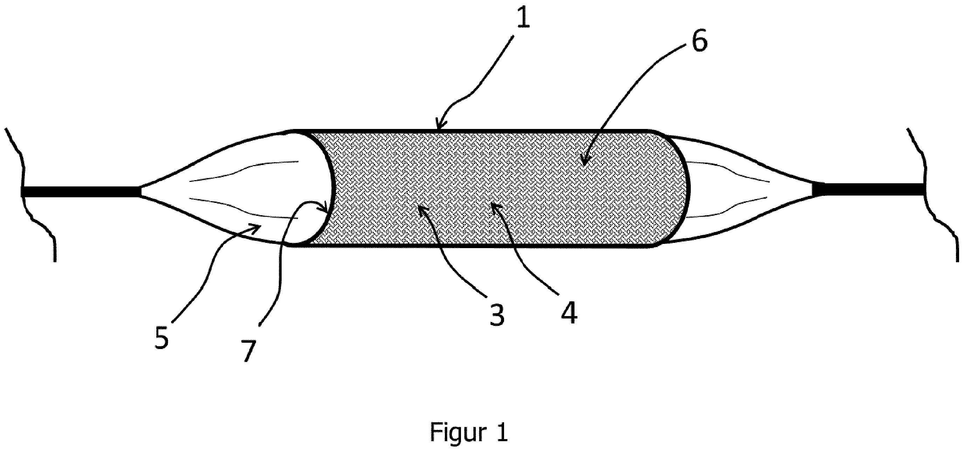

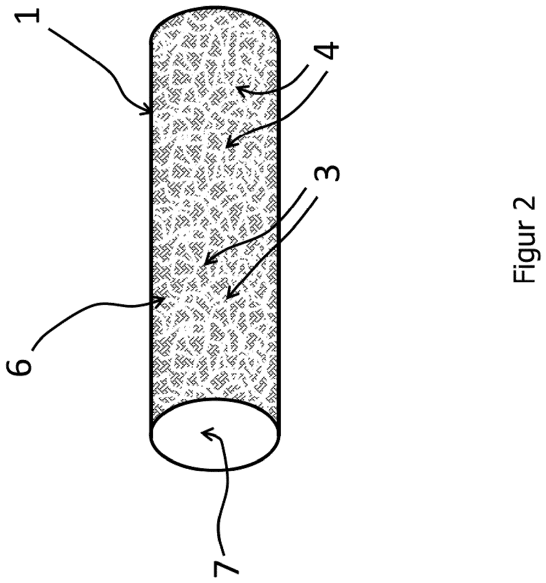

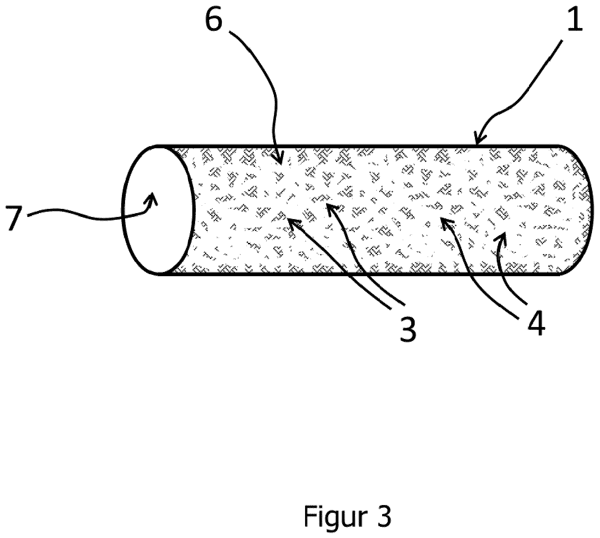

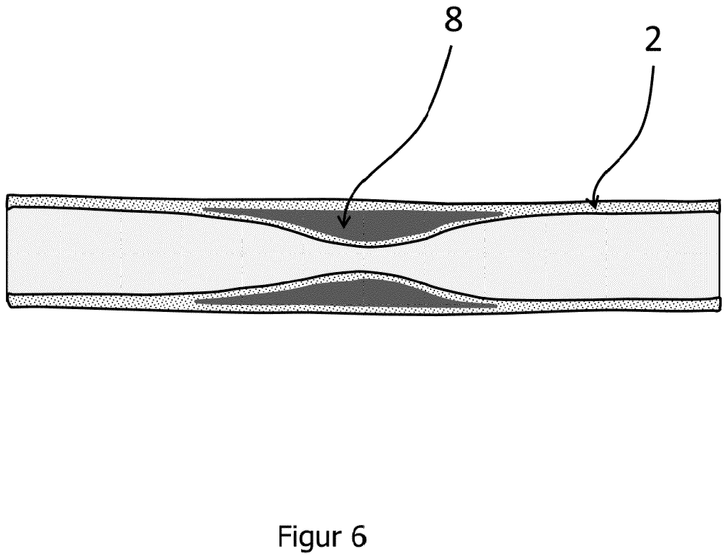

Erfindungsgemäß ist eine rohrförmige Vliesstruktur 1 bzw. Hülse als Wirkstoffträger zur atraumatischen Behandlung von Hohlorganen 2, insbesondere für eine Ballondilatation vorgesehen (Figuren 1-3 und 6-9).

Gemäß einem Ausführungsbeispiel ist die rohrförmige Vliesstruktur 1 aus zumindest ersten biodegradierbaren Polymer-Nano-Fasern 3 und zweiten biodegradierbaren Polymer-Nano-Fasern 4 ausgebildet.

Die ersten Polymer-Nano-Fasern 3 sind aus einem langsam degradierbaren Polymer (PL) ausgebildet. Die zweiten Polymer-Nano-Fasern 4 sind aus einem schnell degradierbaren Polymer (PS) ausgebildet.

Im Rahmen der vorliegenden Erfindung kann auch vorgesehen sein, dass die ersten Polymer-Nano-Fasern 3 aus dem schnell degradierbaren Polymer (PS) und die zweiten Polymer-Nano-Fasern 4 aus dem langsam degradierbaren Polymer (PL) ausgebildet sind. Weitere dritte und/oder vierte degradierbare Polymer-Nanofasern können ebenfalls aus dem schnell degradierbaren Polymer (PS) oder aus dem langsam degradierbaren Polymer (PL) ausgebildet sein.

Eine Degradationszeit des langsam degradierbaren Polymers 3 (PL) beträgt in etwa 1 Woche bzw. 2 Wochen bis 3 Monate bzw. 6 Monate. Für die Anwendung der Kontrazeption können die Fasern 3 (PL) derart ausgebildet sein, dass diese über einen langen Zeitraum von einem Jahr bis 5 Jahre insbesondere bis drei Jahre degradieren. Eine Degradationszeit des schnell degradierbaren Polymer 4 (PS) beträgt in etwa 1 Sekunde bzw. 30 Sekunden bis 1 Minute bzw. bis 5 Minuten bzw. bis 10 Minuten oder bis zu einer Woche.

Das biokompatible Polymer der Polymer-Nano-Fasern kann aus Polymeren auf Basis von Milchsäure (Polylactid, PLA), Glycolsäure (Polyglycolid, PGA) und ihrer Copolymere (Poly(lactid-co-glycolid), - PLGA), sowie Poly(ε-caprolacton), Polyethylenglycol, Polyethylenoxid, Polysebacinsäure, Po-ly(trime-thylencarbonat), Poly(ethylen-co-vinylacetat), Poly(1,5-dioxepan-2-on), Polyvinylpyrrolidon (PVP), Poly-p-dioxanone (PPDX) sowie deren Verbindungen und Copolymeren oder Mischungen daraus ausgebildet sein, wobei die Polymer-Nano-Fasern vorzugsweise einen Faserdurchmesser im Bereich von 300 bis 2000 nm und insbesondere im Bereich von 500 bis 1000 nm aufweisen.

Die Wanddicke der Vliesstruktur ist kleiner 50 µm, vorzugsweise beträgt die Wanddicke in etwa 10 bis 20 µm.

Die langsam und die schnell degradierbaren Polymer-Nano-Fasern können in der rohrförmigen Hülse in einem Verhältnis von 90 Vol-% zu 10 Vol-% bzw. von 80 Vol-% zu 20 Vol-% bzw. von 70 Vol-% zu 30 Vol-% bzw. von 60 Vol-% zu 40 Vol-% bzw. von 50 Vol-% zu 50 Vol-% bzw. von 40 Vol-% zu 60 Vol-% bzw. von 30 Vol-% zu 70 Vol-% bzw. von 20 Vol-% zu 80 Vol-% bzw. von 10 Vol-% zu 90 Vol-% vorgesehen sein.

Universell einsetzbar und gemäß dieser Ausführungsform vorgesehen ist insbesondere ein Verhältnis von langsam und schnell degradierbaren Polymer-Nano-Fasern von 50 Vol-% zu 50 Vol-% (Figur 4). Bevorzugt für kleine Blutgefäße mit einem Durchmesser von 1-5 mm ist insbesondere ein Verhältnis von schnell zu langsam degradierbaren Polymer-Nano-Fasern in der rohrförmigen Hülse von 70 Vol-% zu 30 Vol-%.

Bevorzugt für große Blutgefäße mit einem Durchmesser von 5-20 mm ist insbesondere ein Verhältnis von schnell zu langsam degradierbaren Polymer-Nano-Fasern in der rohrförmigen Hülse von 30 Vol-% zu 70 Vol-%.

Eine oder beide Polymerkomponenten weise klebrige/adhäsive Eigenschaften, wodurch das Haften an der Hohlorganwand begünstigt wird.

Die Vliesstruktur 1 ist in einem (nicht dargestellten) gefalteten Zustand um eine Hülsenlängsachse um den Ballon des Ballonkatheters 5 herum gefaltet. Die Faltung der Hülse ist als eine, um eine Hülsenlängsachse gerichtete Plissierung ausgebildet.

In einem Endzustand ist die Vliesstruktur 1 zum Anliegen an einer Innenwandung eines Hohlorganes 2 ausgebildet.

Ein medizinischer Wirkstoff (nicht dargestellt) ist in die ersten Polymer-Nano-Fasern 3 eingelagert und in Zwischenräumen zwischen den Polymer-Nano-Fasern angeordnet.

Dadurch, dass die Hülse rohrförmig ausgebildet ist, weist diese eine Außenwandung 6 und eine Innenwandung 7 auf.

Die rohrförmige Hülse kann verschiedenste Durchmesser und Längen aufweisen, abgestimmt auf die Geometrie der zu behandelnden Gefäßläsion. Beispielsweise kann eine Ausführung mit einem Durchmesser von 5 mm im expandierten Endzustand und einer Länge von 40 mm eine Wandstärke von 20-30 µm aufweisen.

Im Folgenden wird ein Verfahren zur Herstellung einer rohrförmigen Hülse zur atraumatischen Behandlung von Hohlorganen, insbesondere zur Implantation durch eine Ballondilatation, beschrieben. Das Verfahren umfasst die folgenden Schritte:
Bereitstellen eines Gemisches aus zumindest einem in einem Lösungsmittel gelösten Polymer und einem medizinischen Wirkstoff,
schichtweises Auftragen des Gemisches auf einen zylindrischen Träger, um ein rohrförmiges Vlies aus Polymer-Nano-Fasern auszubilden,
Faltung des Vlieses nach Entfernung des Trägers und gleichsinnige Wicklung der Falten um eine zentrale Achse,
Aufbringung des gefalteten Vlieses auf die gefaltete und gewickelte Ballonmembran eines Ballonkatheters oder
schichtweises Auftragen des Gemisches zur Ausbildung eines rohrförmigen Vlieses aus Polymer-Nano-Fasern direkt auf den aufgedehnten Ballon eines Ballonkatheters, wobei der Ballon vorher mit einer Trennschicht versehen wurde,
und Faltung und gleichsinnige Wicklung des Vlieses zusammen mit der deflatierten Ballonmembran um den Ballonkatheterschaft.

Die einzelnen Fasern verkleben während des Herstellvorgangs miteinander: Zusammenhalt, Formtreue und mechanische Eigenschaften ergeben sich aus der Gesamtkonsistenz der aneinanderhaftenden multiplen Einzelfasern.

Bei der Herstellung durch Sprühen (Air Spraying) oder durch Verspinnen in einem elektrischen Feld (Electrospinning) oder durch eine Kombination davon (Electrostatic Air Spraying) eignen sich als Lösungsmittel Substanzen mit ausreichend hohem Dampfdruck wie z. B. Chloroform, Aceton, Methanol, Heptan, Tetrahydrofuran, und weitere.

Ein erstes langsam degradierbares Polymer kann in einem ersten Lösungsmittel, bzw. einem Lösungsmittelgemisch aus zwei oder mehr Lösungsmitteln, gelöst sein, und ein zweites schnell degradierendes Polymer kann in dem ersten oder in einem zweiten Lösungsmittel, bzw. einem zweiten Lösungsmittelgemisch aus zwei oder mehr Lösungsmitteln, gelöst sein.

Dem Gemisch kann ein Schutzkolloid hinzugefügt sein, dass die Durchmischung verbessert und verhindert, dass sich der wasserunlösliche medizinische Wirkstoff abscheidet, wobei das Schutzkolloid vorzugsweise das schnelllösliche Polymer ist.

Das Auftragen der Lösung auf den zylindrischen Träger kann durch Versprühen mit einem Luftstrahl (Air Spraying) oder durch Verspinnen in einem elektrischen Feld (Electrospinning) oder durch eine Kombination davon (Electrostatic Air Spraying) und/oder durch Tauchen in eine Lösung (Dip Coating) und/oder durch Aufbringen eines kontinuierlichen Schmelzestrangs (Melt Electrospinning Writing) erfolgen oder diskontinuierlich mittels 3D-Druck (additive manufacturing) aufgetragen werden. Normalerweise ist ein Verfahren zum Auftragen der Lösung vorgesehen. Jedoch, sofern technisch sinnvoll, können auch Kombinationen der genannten Verfahren möglich sein.

Auf den Träger wird vor dem Auftragen des Gemisches eine Trennschicht aufgebracht. Als Trennschicht eignen sich z.B. hämokompatible, leicht wasserlösliche Polymere und Zuckerverbindungen.

Der Wirkstoff kann auch gemischt in einer Polymer (langsam degradierend - PL)-Lösungsmittel-Lösung per Tauchverfahren (Dip Coating) außen auf das Vlies auftragen werden. Nach Verdampfen des Lösungsmittels lagert sich das Polymer PL mit dem Wirkstoff an die Fasern des Vlieses an, wodurch die Mikrokompartimente zwischen den Fasern teilweise gefüllt werden. Das Vlies kann dabei vorzugsweise rein aus schnell degradierendem Polymer PS oder einem PL/PS-Gemisch bestehen.

Vor dem Falten können intermittierende Längsschnitte in die Hülse eingebracht werden. Die Faltung des Vlieses kann durch intermittierende Längsschlitze im Verlauf der Faltkanten erleichtert werden. Diese Längsschlitze können vorzugsweise durch Laserschnitt erzeugt werden.

Der Träger ist ein zylindrischer Trägerkörper oder ein inflatierter Ballon sein.

Bevorzugt ist der direkte Auftrag auf einen Ballon. Dazu wird auf die aufgeblasene (inflatierte) Ballonhülle eines Ballonkatheters zunächst eine Trennschicht aufgetragen. Auf die Trennschicht wird dann die Hülse aufgetragen.

Die rohrförmige Hülse wird zusammen mit dem Ballon des Ballonkatheters um den Katheterschaft gefaltet.

Insbesondere ist erfindungsgemäß ein System zur atraumatischen Behandlung von Hohlorganen vorgesehen, das einen Ballonkatheter und die erfindungsgemäße rohrförmige Hülse umfasst.

Zudem kann bei diesem System auf der Hülse im Ausgangszustand eine äußere Schutzhülle angeordnet sein.

Die erfindungsgemäße rohrförmige Hülse ist auf einen Ballon eines Ballonkatheters aufgebracht. Durch Aufdehnung des Ballons im Hohlorgan (speziell: Blutgefäß) wird sie abgeworfen und haftet an der Hohlorganwand (speziell: Gefäßwand). Die Hülse wird somit durch die Ballonentfaltung im Hohlorgan (speziell: Blutgefäß) mit ihrer Außenwandung gegen die Hohlorganwand (speziell: Gefäßwand) gepresst. Dadurch entsteht während des Abwurfs ein flächiger Kontakt der gesamten Außenwandung der Hülse mit Körperflüssigkeit (speziell: Blut), und nach Abwurf und Ballondeflatation ein flächiger Kontakt der Innenwandung mit Körperflüssigkeit (Blut), wobei die Außenwand in flächigem Kontakt mit der Hohlorganwand (Gefäßwand) steht. Da die Hülse mit einem medizinischen Wirkstoff beladen ist, diffundiert der Wirkstoff in die Hohlorganwand (Gefäßwand). Die Wirkstoffabgabe erfolgt solange, bis die Hülse biologisch abgebaut worden ist.

Weiterhin kann die Hülse derart ausgebildet sein, dass durch eine "Liner"-Funktion die bei Ballondilatation entstehenden Längsrisse in der Wand abgedeckt werden, zum Beispiel, um damit im Blutgefäß die Thrombogenität zu reduzieren. Einrisse in der Wandung des Hohlorganes, die durch die Expansion bzw. die Inflation des Ballonkatheters entstanden sind, können durch die entfaltete, die Läsion komplett abdeckende Hülse von direktem Kontakt mit Körperflüssigkeit (Blut) ausgeschlossen werden. Auf diese Weise wird bei der Applikation im Blutgefäß die Thrombogenität, d. h. die Neigung zur Blutgerinnselbildung im Bereich der behandelten Gefäßläsion vermindert.

Erfindungsgemäß ist somit eine aus multiplen Mikro-Nano-Fasem gebildete rohrförmige Hülse auf einem Ballon eines Ballonkatheters anordbar und ist vorzugsweise zusammen mit dem Ballon des Ballonkatheters faltbar und aufdehnbar, wobei sich die Hülse bei Ballonaufdehnung vom Ballon löst und an die Hohlorganwand gepresst wird, und nach Ballondeflatierung an der Hohlorganwand haftet, und den Wirkstoff eingeschlossen in einer langsam degradierbaren Faserkomponente (PL) beinhaltet, die im Verlaufe ihres biologischen Abbaus (Degradation) den therapeutisch wirksamen Wirkstoffspiegel für einen kritischen Zeitraum im Kontakt zu der Hohlorganwand liefert, und gleichzeitig auch die nach Ballondilatation entstandenen Längsrisse in der Hohlorganwand abdichtet. Auf diese Weise lässt sich die Wirkstoffabgabe und auch deren Konzentration über die Zeit optimal auf den Anwendungsfall einstellen.

Dadurch, dass gemäß einer bevorzugten Ausführungsform besonders schnell bioabbaubare Komponenten vorgesehen sind, beginnt nach dem Abwurf des Ballons und der Anhaftung an der Hohlorganwand ein schneller Zerfall der Hülse in multiple Platten bzw. Plättchen bzw. Mikroflakes in einer insgesamt hülsenförmigen Anordnung.

Somit zeichnet sich diese Ausführungsform dadurch aus, dass die schnelle Auflösung der entsprechenden Fasern (innerhalb weniger Sekunden bis zu 5 Minuten) einen Zerfall der vormals konsistent zylindrischen Hülse in multiple "Flakes" (= Mikropartikel bzw. "Microflakes") bewirkt, die flach sind und der Hohlorganwand anhaften.

Dabei bestehen die resultierenden Flakes überwiegend aus der langsam degradierbaren Komponente, die den Wirkstoff enthält, während die schnell degradierbare Komponente durch Auflösung den Zusammenhalt der vormalig zusammenhängenden Hülse als ein einziges Teil aufhebt und den Zerfall der Hülse in multiple Teile ("Microflakes") induziert.

Bevorzugt hat ein einzelnes Flake eine flächige Ausdehnung von 10 bis 30 µm und eine Dicke von 5 bis 10 µm.

Die erfindungsgemäße Vliesstruktur bzw. das erfindungsgemäße System ist für die Folgenden medizinischen und therapeutischen Verfahren vorgesehen.

Die Wirkstoff-Beladung des Wirkstoffträgers hängt ab vom Wirkort (Hohlorgan) und der Erkrankung. Der medizinische Wirkstoff kann einen oder mehrere der folgenden Stoffe umfassen.

Zur Reduzierung einer überschiessenden Wandreaktion (intimale Hyperplasie) in Blutgefäßen, insbesondere in Arterien können als medizinscher Wirkstoff bspw. Limus-Derivate (z.B. Sirolimus) oder Paclitaxel (PTX) vorgesehen sein.

Limus-Derivate haben eine viel günstigere biologische Wirkung als Paclitaxel. Sie lassen sich jedoch schlecht mit herkömmlich beschichteten Ballons per einmaligem kurzen Ballonkontakt in die Gefäßwand übertragen, da sie deutlich schlechter anhaften als Paclitaxel-Kristalle, die in die Gefäßwand gespießt werden. Zudem benötigen sie wesentlich mehr Kontaktzeit für eine wirksame Übertragung in die Gefäßwand. Im Falle der abgeworfenen Hülse entspricht die Kontaktzeit mit der Gefäßwand der Lebendauer der Hülse, bis sie degradiert ist. Gleichzeitig ist die transferierte Hülse auch eine Ge-währ für die transferierte Wirkstoffmenge (was bei einmaligem Ballonkontakt mit herkömmliche Sys-temen unwägbar ist) und sie hält den Wirkstoff besser am Wirkort (bei herkömmlicher Ballonübertra-gung rauscht ein großer Teil der übertragenen Partikel mit dem Blutstrom ab in die Peripherie).

Für die Anwendung im Blutgefäßsystem soll die implantierte Hülse somit insbesondere als Träger für Antiproliferativa wie Limus-Derivate dienen und damit deren komplette Übertragung mit einem längerfristigen Kontakt zur Gefäßwand gewährleisten. Das Hauptanwendungsgebiet liegt im Bereich der Arterien, insbesondere in Ober und Unterschenkelbereich, aber auch in den Herzkranzgefäßen. Während die Oberschenkelgefäße noch Durchmesser bis zu 7 mm aufweisen liegen die Unterschenkelgefäße und die Herzkranzgefäße mit ihren Aufzweigungen bei 5-2 mm Durchmesser. Denkbar ist auch eine Anwendung im Venensystem oder bei Hämodialyseshunts.

Für die Anwendung als Eileiter-Wirkstoffträger mit kontrazeptiver Langzeitwirkung können als medizinsche Wirkstoffe langzeitstabile Depot-Gestagene, wie z.B. Etonogestrel, Levonorgestrel oder ein geeignetes Antiprogesteron, wie z.B. Mifepriston oder ein Spermizid, wie z.B. Nonoxinol 9 vorgesehen sein.

Zur Therapie von Karzinomen in Hohlorganen, z.B. in den Gallenwegen können für die Hülse als medizinscher Wirkstoff Zytostatika wie z.B. Mitomycin, Capecitabin oder Methotrexat (MTX) vorgesehen sein.

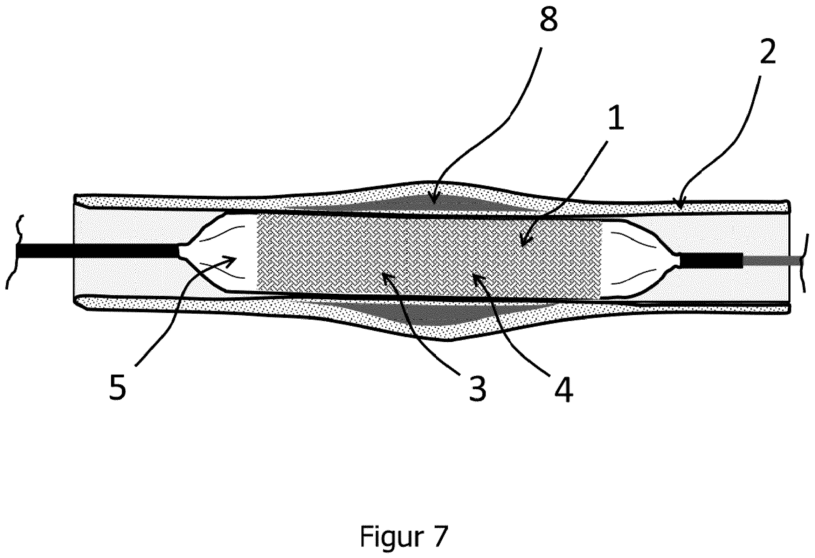

Die Vliesstruktur bleibt nach der Herstellung für den Zeitraum der Lagerung des Ballonkatheters auf der Ballonmembran bis zur Applikation komplett und vollständig (= konsistent), mit einer zusammenhängenden zylindrischen Innen- und Außenfläche (Figuren 1 und 7).

Im Moment der Ballonentfaltung bei der Anwendung im Patienten entsteht ein flächiger Kontakt der gesamten Außenfläche der Hülse (und nach Abwurf auch der Innenfläche) mit Körperflüssigkeit bzw. Blut.

Die Hülse wird durch die Ballonentfaltung im Hohlorgan (speziell: Blutgefäß) mit ihrer Außenfläche gegen die Hohlorganwand (speziell: Gefäßwand) gepresst (Figur 7).

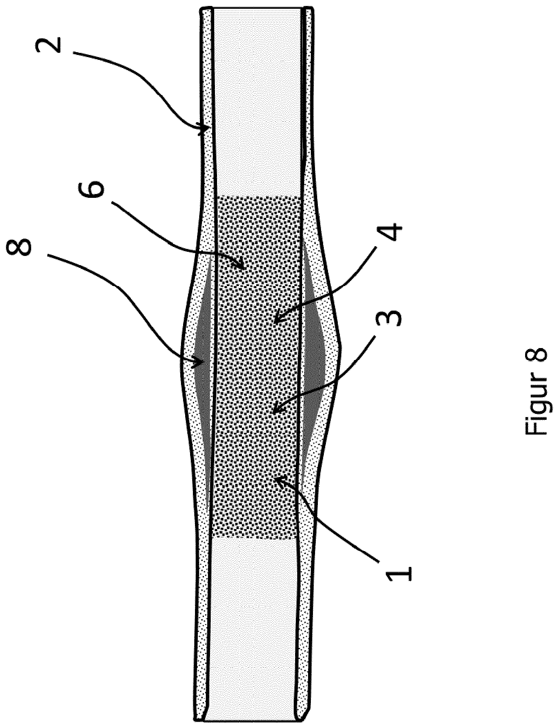

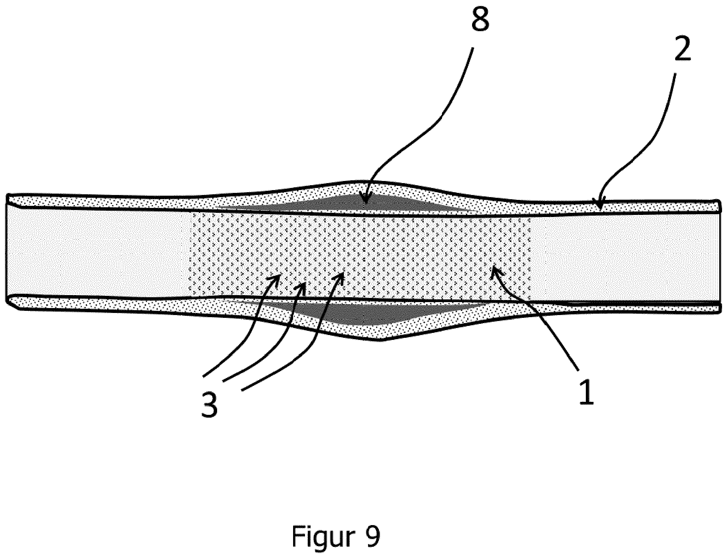

Bei Deflation des Ballons haftet die Hülse mit Ihrer Außenfläche an der Hohlorganwand (aufgrund des klebrig-adhäsiven Materialcharakters) und es entsteht durch komplette Ablösung der Hülse von der Ballonmembran ein flächiger Kontakt der gesamten Innenfläche der Hülse mit Körperflüssigkeit bzw. Blut (Figuren 8 und 9)

Die Haftung an der Gefäßwand kann durch eine radiale Eigensteifigkeit der Hülse nach Entfaltung verstärkt werden.

Die gleichmäßige Ablösung von der Ballonmembran kann durch vorheriges Auftragen einer Trennschicht auf die Ballonmembran verbessert werden.

Die Benetzung der Innen- und Außenfläche mit Körperflüssigkeit bzw. Blut kann die Auflösung der Hülse (= Biodegradation, Abbau im Körper) induzieren, sofern die Hülse aus bioabbaubaren Polymeren besteht.

Für die Anwendung im Blutgefäß ist der Wirkstoff bevorzugt ein Antiproliferativum wie Sirolimus, oder andere Limus-Derivate oder Paclitaxel.

Für die Anwendung als Eileiter-Wirkstoffträger mit kontrazeptiver Langzeitwirkung ist der Wirkstoff bevorzugt ein langzeitstabiles Depot-Gestagen, wie z.B. Etonogestrel, Levonorgestrel oder ein geeignetes Antiprogesteron, wie z.B. Mifepriston oder ein Spermizid, wie z.B. Nonoxinol 9 vorgesehen sein, oder eine Kombination daraus.

Für die Anwendung als Karzinom-Therapie in Hohlorganen, z.B. in den Gallenwegen, ist der Wirkstoff bevorzugt ein Zytostatikum, wie z.B. Mitomycin, Capecitabin oder Methotrexat (MTX).

Bei Abwurf der Hülse vom Ballonkatheter induziert der Kontakt von Außen- und Innenfläche mit Körperflüssigkeit oder Blut den Degradationsprozess.

Im Falle der schnell degradierbaren Komponente kommt es zu einer schnellen Auflösung. Diese schnelle Auflösung (innerhalb weniger Sekunden bis zu 5 Minuten) kann einen Zerfall der vormals konsistent zylindrischen Hülse in multiple "Flakes" (= Mikropartikel bzw. "Microflakes") bewirken, die flach sind und der Hohlorganwand anhaften (z.B. Mikroskopiebild Figur 5).

Die Größe der Flakes hängt ab von der Degradationskinetik der beiden Polymere und von deren Mischungsverhältnis. Die genaue Degradationskinetik im Blut oder in anderen Körperflüssigkeiten lässt sich einstellen und hängt ab von der Art des Polymers, von dem Molekulargewicht und der Art der Seitenketten (hydrophil oder hydrophob).

Die Vliesstruktur kann zusätzlich und/oder alternativ zu den vorstehend genannten Merkmalen die Folgenden Merkmale aufweisen.

Die in etwa polygonalen Zwischenräume bzw. Mikrokompartimente zwischen den einzelnen Polymer-Nano-Fasem weisen maximal eine Weite kleiner 100 µm, bzw. kleiner 80 µm, bzw. kleiner 60 µm, bzw. kleiner 40 µm, bzw. kleiner 30 µm bzw. kleiner 10 µm und vorzugsweise kleiner 20 µm auf. Die angegebenen Werte können sich auch auf eine maximale Erstreckung in Längsrichtung eines elliptischen oder andersförmig ausgebildeten Zwischenraumes beziehen, wobei die Werte dann die breiteste Weite der Zwischenräume betreffen.

Die rohrförmige Hülse kann auch mit einer Hydrogelschicht, zur Haftverbessung dotiert sein. Die Hydrogelschicht weist bevorzugt eine Dicke von ca. 5 µm bis 20 µm, bzw. von 7,5 bis 12,5 µm und insbesondere von 10 µm auf.

Durch das Dotieren der Außenfläche der Hülse mit einer Hydrogelschicht klebt die Hülse aufgrund der Hydrogelschicht nach der Entfaltung an einer Innenwandung eines Hohlorganes, insbesondere eines Blutgefäßes, an.

Zudem kann ein biokompatibler Kleber, wie z.B. ein Hydrogel, den Faltungszustand der Hülse während des Transports auf dem Ballonkatheter zum Implantationsort stabilisieren.

Ein Vlies ist ein Gebilde aus Fasern begrenzter Länge, Endlosfasern (Filamenten) oder geschnittenen Garnen jeglicher Art und jeglichen Ursprungs, die auf irgendeine Weise zu einem Vlies (einer Faserschicht, einem Faserflor) zusammengefügt und auf irgendeine Weise miteinander verbunden worden sind.

Vliesstoffe sind größtenteils flexible textile Flächengebilde, d. h. sie sind leicht biegsam, ihre Hauptstrukturelemente sind textile Fasern und sie weisen eine vergleichsweise geringe Dicke gegenüber ihrer Länge und Breite auf. Ebenso existieren Vliesstoffe, die wegen der verwendeten Fasern oder der Verfestigungsverfahren eher Papieren, Folien oder faserverstärkten Kunststoffen als Textilien ähneln. Vliesstoffe stellen eine Materialgruppe mit einer großen Eigenschaftsvielfalt dar, die einem breiten Spektrum von medizinischen Anwendungsanforderungen gezielt angepasst werden kann.

Weiterhin kann die Hülse zylindrisch ausgebildet sein. Alternativ kann die Hülse auch jede aus dem Stentbereich bekannte Form aufweisen. D.h. die Hülse kann konisch, verzweigt, eingeschnürt (nach Art einer Eieruhr), elliptisch oder kreisförmig in einer Seitenansicht ausgebildet sein.

Das Material, aus dem die Hülse ausgebildet ist, und ihre Beschichtung weisen vorzugsweise eine gewisse Anschmiegsamkeit bzw. Anlegbarkeit auf, um sich an die Struktur einer Wandung bzw. die Oberflächenbeschaffenheit eines Hohlorganes anzupassen und auf dies Weise Läsionen abzudecken.

Weiterhin kann eine Dotierung der Innenfläche der Hülse mit einer antithrombogenen Substanz, wie z.B. Heparin, und bzw. oder einem Wirkstoff erfolgen, der eine Endothelzellen-Besiedlung fördert.

Die Haftung der Substanzen kann durch Oberflächenstrukturierung oder Gelfilm-Auflagerung der Oberflächen der Hülse verbessert werden.

### Bezuqszeichenliste

1 rohrförmige Vliesstruktur
2 Hohlorgan
3 erstes degradierbares Polymer
4 zweites degradierbares Polymer
5 Ballon des Ballonkatheters
6 Außenwandung
7 Innenwandung
8 Engstelle

## Patentansprüche

1. Rohrförmige Vliesstruktur als Wirkstoffträger zur atraumatischen Behandlung von Hohlorganen, insbesondere für eine Applikation über einen Ballonkatheter, wobei die Hülse in einem Ausgangszustand um eine Hülsenlängsachse gefaltet ist und in einem Endzustand zum Anliegen an einer Innenwandung eines Hohlorganes entfaltbar ist,
wobei die rohrförmige Hülse aus zumindest ersten biodegradierbaren Polymer-Nano-Fasern ausgebildet ist und die Faltung der Hülse als Plissierung um eine Hülsenlängsachse gerichtet ist, wobei ein medizinischer Wirkstoff in die ersten Polymer-Nano-Fasern eingelagert und/oder in Zwischenräumen zwischen den Polymer-Nano-Fasern angeordnet ist, und wobei die ersten Polymerfasern derart ausgebildet sind, dass die Polymerfasern über einen einstellbaren Zeitraum von 2 Wochen bis 3 Monaten degradieren, sodass der Wirkstoff in diesem Zeitraum an eine Hohlorganwandung abgebbar ist.

2. Rohrförmige Hülse gemäß Anspruch 1,
**dadurch gekennzeichnet,**
**dass** die rohrförmige Hülse aus zumindest den ersten und zweiten, vorzugsweise biodegradierbaren, Polymer-Nano-Fasern ausgebildet ist.

3. Rohrförmige Hülse gemäß Anspruch 1 oder 2,
**dadurch gekennzeichnet,**
**dass** die zweiten biodegradierbaren Polymer-Nano-Fasern oder weitere Polymer-Nano-Fasern derart ausgebildet sind, dass die Polymer-Nano-Fasern über einen einstellbaren Zeitraum von 1 Sekunde bis 2 Wochen degradieren.

4. Rohrförmige Hülse gemäß einem der Ansprüche 1 bis 3,
**dadurch gekennzeichnet,**
**dass** die langsam und die schnell degradierbaren Polymer-Nano-Fasern in der rohrförmigen Hülse in einem Verhältnis von 90 Vol-% zu 10 Vol-% bzw. von 80 Vol-% zu 20 Vol-% bzw. von 70 Vol-% zu 30 Vol-% bzw. von 60 Vol-% zu 40 Vol-% bzw. von 50 Vol-% zu 50 Vol-% bzw. von 40 Vol-% zu 60 Vol-% bzw. von 30 Vol-% zu 70 Vol-% bzw. von 20 Vol-% zu 80 Vol-% bzw. von 10 Vol-% zu 90 Vol-% vorgesehen sind.

5. Rohrförmige Hülse gemäß einem der Ansprüche 1 bis 4,
**dadurch gekennzeichnet,**
**dass** das biokompatible Polymer der Polymer-Nano-Fasern aus Polymeren auf Basis von Milchsäure (Polylactid, PLA), Glycolsäure (Polyglycolid, PGA) und ihrer Copolymere (Poly(lactid-co-glycolid), PLGA), sowie Poly(ε-caprolacton), Polyethylenglycol, Polyethylenoxid, Polysebacinsäure, Poly(trime-thylencarbonat), Poly(ethylen-co-vinylacetat), Poly(1,5-dioxepan-2-on), Polyvinylpyrrolidon (PVP), Poly-p-dioxanone (PPDX) sowie deren Verbindungen und Copolymeren oder Mischungen daraus ausgebildet ist, wobei die Polymer-Nano-Fasern vorzugsweise einen Faserdurchmesser im Bereich von 300 bis 2000 nm und insbesondere im Bereich von 500 bis 800 nm aufweisen.

6. Rohrförmige Hülse gemäß einem der Ansprüche 1 bis 5
**dadurch gekennzeichnet,**
**dass** der medizinische Wirkstoff ein Antiproliferativum, bspw. Sirolimus, oder andere Limus-Derivate oder Paclitaxel (PTX) oder langzeitstabile Depot-Gestagene, bspw. Etonogestrel, Levonorgestrel oder ein Antiprogesteron, bspw. Mifepriston oder ein Spermizid bspw. Nonoxinol 9 oder Zytostatika, bspw. Mitomycin, Capecitabin oder Methotrexat (MTX) umfasst.

7. Rohrförmige Hülse gemäß einem der Ansprüche 1 bis 6,
**dadurch gekennzeichnet,**
**dass** die rohrförmige Hülse eine radiale Stützschicht aufweist, wobei radiale Stützschicht durch Polymer-Nano-Fasern mit höherer Festigkeit und/oder durch eine zusätzliche Polymerschicht ausgebildet ist (z.B. durch ein lasergeschnittenes tubuläres, degradierbares Polymer-Halbzeug, oder durch eine mittels Melt Electrospinning Writing - MEW gebildete Schicht).

8. Rohrförmige Hülse gemäß einem der Ansprüche 1 bis 7,
**dadurch gekennzeichnet,**
**dass** zumindest eine äußere Mantelwandung der Hülse adhäsive Eigenschaften aufweist und/oder mit einer Beschichtung derart versehen ist, dass die Mantelwandung beim Entfalten an einer Innenwandung eines Hohlorganes anhaftet.

9. Verfahren zur Herstellung einer rohrförmigen Hülse zur atraumatischen Behandlung von Hohlorganen, insbesondere für eine Ballondilatation, umfassend die folgenden Schritte:
Bereitstellen eines Gemisches aus zumindest einem in einem Lösungsmittel gelösten Polymer und einem medizinischen Wirkstoff,
schichtweises Auftragen des Gemisches auf einen zylindrischen Träger, um ein rohrförmiges Vlies aus Polymer-Nano-Fasern auszubilden,
Faltung und gleichsinnige Wicklung des Vlieses um die Längsachse nach Entfernung des Trägers und Aufbringen des Vlieses auf einen Ballon eines Ballonkatheters,
oder schichtweises Auftragen des Gemisches direkt auf einen entfalteten Ballon eines Ballonkatheters auf den vorher eine Trennschicht aufgebracht wurde, und
vorzugsweise Faltung und Wicklung zusammen mit der Ballonmembran des Ballonkatheters nach Deflation des Ballons.

10. Verfahren gemäß Anspruch 9,
**dadurch gekennzeichnet,**
**dass** das Auftragen der Lösung auf den zylindrischen Träger oder direkt auf den Ballon durch Versprühen mit einem Luftstrahl (Air Spraying) oder durch Verspinnen in einem elektrischen Feld (Electrospinning) und/oder durch eine Kombination davon (Electrostatic Air Spraying) und/oder durch Tauchen in eine Lösung (Dip Coating) und/oder durch Aufbringen eines kontinuierlichen Schmelzestrangs (Melt Electrospinning Writing) erfolgt oder diskontinuierlich mittels 3D-Druck aufgetragen wird.

11. Verfahren gemäß Anspruch 9 oder 10,
**dadurch gekennzeichnet,**
**dass** für die Herstellung durch Sprühverfahren oder Electrospinning als Lösungsmittel Substanzen mit ausreichend hohem Dampfdruck wie z. B. Chloroform, Aceton, Methanol, Heptan, Tetrahydrofuran, und weitere vorgesehen sind.

12. Verfahren gemäß einem der Ansprüche 9 bis 11,
**dadurch gekennzeichnet,**
**dass** ein erstes langsam degradierbares Polymer in einem ersten Lösungsmittel, bzw. einem Lösungsmittelgemisch aus zwei oder mehreren Lösungsmitteln, gelöst ist, und ein zweites schnell degradierendes Polymer in dem ersten oder in einem zweiten Lösungsmittel, bzw. einem zweiten Lösungsmittelgemisch aus zwei oder mehreren Lösungsmitteln, gelöst ist.

13. Verfahren gemäß einem der Ansprüche 9 bis 12,
**dadurch gekennzeichnet,**
**dass** dem Gemisch ein Schutzkolloid hinzugefügt ist, das die Durchmischung verbessert und verhindert, dass sich der wasserunlösliche medizinische Wirkstoff abscheidet, wobei das Schutzkolloid vorzugsweise das schnelllösliche Polymer ist.

14. Verfahren gemäß einem der Ansprüche 9 bis 13,
**dadurch gekennzeichnet,**
**dass** auf den Träger vor dem Auftragen des Gemisches eine Trennschicht aufgetragen wird.

15. Verfahren gemäß einem der Ansprüche 9 bis 14,
**dadurch gekennzeichnet,**
**dass** der Träger ein zylindrischer Trägerkörper oder ein inflatierter Ballon ist.
Bevorzugt ist der direkte Auftrag auf einen Ballon. Dazu wird auf die aufgeblasene (inflatierte) Ballonhülle eines Ballonkatheters zunächst eine Trennschicht aufgetragen. Auf die Trennschicht wird dann die Hülse aufgetragen.
